Europäisches Patentamt

European Patent Office

Office européen des brevets

(19)

(11) Publication number: **0 148 749**
**A2**

# (12) EUROPEAN PATENT APPLICATION

(21) Application number: **85100076.0**

(22) Date of filing: **04.01.85**

(51) Int. Cl.⁴: **A 24 B 15/16,** A 61 K 7/16,
A 61 K 47/00

(30) Priority: **09.01.84 US 569280**

(43) Date of publication of application: **17.07.85**
**Bulletin 85/29**

(84) Designated Contracting States: **AT BE CH DE FR GB IT**
**LI LU NL SE**

(71) Applicant: **ADVANCED TOBACCO PRODUCTS, INC.,**
**One River Walk Place, Suite 402 700 North St. Mary's**
**Street, San Antonio Texas 78205 (US)**

(72) Inventor: **Ray, Jon Philip, 12544 Judson Road, San**
**Antonio Texas 78233 (US)**
Inventor: **Thyagarajan, B.S.,**
**12711 Interstate 10 Apartment 1914, San Antonio**
**Texas 78230 (US)**

(74) Representative: **Patentanwälte Grünecker, Dr.**
**Kinkeldey, Dr. Stockmair, Dr. Schumann, Jakob, Dr.**
**Bezold, Meister, Hilgers, Dr. Meyer-Plath,**
**Maximilianstrasse 58, D-8000 München 22 (DE)**

(54) **Nicotine preparation.**

(57) This invention provides a nicotine preparation having utility in combination with dispensing devices. Specifically, the nicotine preparation of this invention includes nicotine, a nicotine enhancing material, an organic acid and a volatile carrier. Alternate embodiments of the invention include flavoring material and/or water.

# NICOTINE PREPARATION

The administration of nicotine can give satisfaction in the usual method of smoking, either cigarette smoking, cigar smoking, or pipe smoking. However, smoking may have health hazards and so it would be desirable to formulate an alternative manner of administering nicotine in a pleasurable manner than can be used to facilitate withdrawal from smoking and/or as a replacement for smoking.

Individuals using tobacco frequently desire to terminate its use for health, social, or economic reasons. It is well known that the smoking, chewing or snuffing of tobacco for protracted periods makes the termination of its use difficult and in some instances almost impossible.

Many preparations have been devised to combat tobacco habits. These prior efforts have generally been directed to the concurrent use of tobacco and an emetic or other substance causing nausea or ill feeling to the end of building up a psychological response to tobacco that makes the same undesirable effect through its continued association with an undesirable habit. Not only have such preparations proved unpopular because of the lack of desire on the part of the individual deliberately to make himself ill to cure the habit, but has proved ineffective in many instances even when faithfully employed.

Despite this, the use of nicotine has been practiced by persons in many cultures, who derive satisfaction from the substance. Nicotine is a toxic liquid alkaloid having the formula $C_5H_4NC_4H_7NCH_3$. When the nicotine is obtained from tobacco, as by chewing, sniffing, or smoking the substance, the amount of nicotine absorbed into the body generally does not build up to a harmful dose, but produces certain pleasurable effects, frequently leading to habitual use.

Nicotine is an alkaloid, such as caffeine, morphine, quinine, codeine, cocaine, etc. The effective pleasure dosage required by the human central nervous system is extremely small. Approximately 50 micrograms are contained in a typical cigarette puff which the user considers satisfying. But, for example, several milligrams of caffeine are required to satisfy a coffee drinker. Also, unlike other alkaloids, free base nicotine is a volatile liquid, which vaporizes easily. Pure nicotine is also characterized as a colorless, transparent liquid, as opposed to the commonly perceived brown, gummy substance.

One of the most popular versions of nicotine use involves the smoking of cigarettes. When the tobacco in a conventional cigarette is ignited, the combustion of the processed tobacco within the cigarette causes the release of vaporous nicotine, which is drawn through the cigarette and into the user's mouth and lungs, when the user sucks or inhales air through the cigarette.

Although nicotine can thus be readily introduced into the body through cigarette smoking, the combustion of the tobacco, with the consequent elevated temperatures required in this process, unfortunately result in a number of undesirable consequences associated with smoking combustible cigarettes. Of primary concern are the serious health hazards known to result from smoking combustible cigarettes. Although the nicotine content of a cigarette is not believed to cause any serious adverse long term health effects on the human body, many other components which are harmful are present in tobacco smoke. Some of these other constituents are known carcinogens, for example. A table listing some of the harmful components in tobacco smoke may be found on pp. 496-501 of the publication Tobacco and Tobacco Smoke, Studies in Experimental Carcinogenesis (1967) by Ernest L. Wynder and Dietrich Hoffman of the Sloan-Kettering Institute for Cancer Research. The teaching of that publication is hereby incorporated by reference into this application.

Furthermore, the smoking of combustible cigarettes may pose a significant fire hazard. Many fires which have occurred both within buildings and in natural environments are attributable to burning cigarettes which were carelessly discarded. In addition, substantial economic losses can be attributed to smoking, including significant damage to business and personal property resulting from burns in clothing, carpeting, furniture, etc. caused by stray ashes from cigarettes. Cigarette smoking has also become increasingly objectionable because of the discomfort it may cause nonsmokers who are exposed to the smoke and odor produced by the smoking habit.

Because of these undesirable side effects of combustible cigarette smoking, attempts have been made from time to time to provide an acceptable substitute for combustible cigarette smoking which will eliminate or ameliorate the adverse consequences mentioned above. Tobacco concentrates, for example, have been processed into a tablet form which may be sucked or chewed in the mouth of the user, at least a portion of the nicotine being absorbed into the user's body.

Various other smoking substitutes have been developed which include cigarette simulating devices containing various materials which approximate the taste and aroma of tobacco or release various other additional aromatic vapors which are intended to have a satisfying effect on the user when those vapors are inhaled. In one such device, synthetic materials simulating the taste and aroma of tobacco are micro-encapsulated within a cigarette substitute device. The desired vapors are released by squeezing or crushing the device, causing the capsules to burst and the vapors to be released into the air drawn through the device. In another such device, the flavor and taste components of tobacco are saturated within a capsule containing an absorbent material, and, when punctured, the capsule releases the aroma and flavor volatiles of tobacco into the air drawn through the device. These devices, however, have failed to take into account that the primary physiological phenomenon related to cigarette smoking, which must be provided in any effective substitute, is the rapid intake of nicotine vapor into the user's lungs to satisfy the user's habit.

In virtually all previous attempts to design devices to alleviate the smoking habit, the inventor has consistently attempted to remove nicotine from the inhalant. This is precisely opposite the intended effect of the use of the nicotine preparation of this invention. This invention is designed to satisfy the desire for nicotine without requiring exposure to adverse pyrolytic by-products.

Thus, despite the various attempts which have been made to provide effective substitutes for combustible cigarettes, no one has developed a device which permits the user to in-

0148749

hale controlled amounts of nicotine vapor, free of all known or suspected carcinogens, sufficient to satisfy a nicotine habit without the need for combustion or other heating means and without the need for the user taking some unfamiliar action other than the actions performed in the conventional smoking habit, namely drawing or sucking a gaseous mixture into the lungs of the user. Certain devices and improved devices are described in commonly-assigned U.S. Patent 4,284,089 as well as in commonly assigned, co-pending patent applications entitled "Improved Nicotine Dispensing Device" and "Nicotine Mixture," both patent applications having been filed on even date herewith.

To this end, it was deemed advisable to develop nicotine formations which can be used in the various devices found in the prior art. In many of these devices, it is necessary for a liquid or fluid nicotine formulation to be present for application to the user by various means. Certain prior art devices seek to supply the nicotine as a chewable smoking substitute. See for instance U.S. Patent 3,877,568 (1975). In addition, others have attempted to incorporate different materials to enhance nicotine in order to make it more acceptable to the body. See for instance U.S. Patent 2,600,700 (1952). In this particular patent, the desired effect is to precondition taste buds to be non-responsive to nicotine.

Other prior art patents disclose the use of nicotine as an ingredient in an insecticide. U.S. Patent 1,776,862 (1932) to Lindstaedt recites the use of nicotine in an insecticide. Similarly, U.S. Patent 2,139,839 (1938) to McKinney and U.S. Patent 2,775,591 (1956) to Creg et al. Also disclosed is the use of nicotine in pest control formulas.

Finally, U.S. Patent 3,390,685 (1966) to Van Bethman et al discloses a technique for the extraction of nicotine from tobacco. Thus, the prior art does not teach a nicotine formula such as described in applicants invention.

Therefore, a need has developed for a nicotine preparation which has utility in the nicotine dispensing devices which are commercially available.

Furthermore, a need has arisen for a vaporizable nicotine source for use in the various nicotine dispensing devices available commercially. Most importantly, it has been found advantageous to provide a nicotine preparation wherein the nicotine moiety itself has been increased in size such that it will bypass absorption in the buccal mucosa and instead passes straight into the lungs where it can be absorbed by the blood stream going to the brain more directly.

These and other objects of the present invention will become apparent from the following summary and description of the preferred embodiments.

This invention provides a nicotine preparation having utility in combination with dispensing devices. Specifically, the nicotine preparation of this invention includes nicotine, a nicotine complexing material, an organic acid and a volatile carrier. Alternate embodiments of the invention include flavoring material and/or water.

The nicotine used in the preparation or formulation of this invention is referred to as a vaporizable nicotine mixture and is selected from the group consisting of nicotine (d), nicotine (l), nicotine (dl) and nicotine salts. The vaporizable nicotine mixture may further include a flavoring material such as (but not limited to) orange flavoring, lemon flavoring, menthol, spearmint flavoring, peppermint flavoring, cinnamon flavoring or other ingredients for flavoring. The nicotine mixture may also include water to adjust the volatility of the nicotine vapors released.

Nicotine is obtained by extraction from dried tobacco leaves where it occurs to the extent of 2% to 8%, combined with citric, malic acids and other acids. The commercial nicotine which is available in the marketplace is entirely a by-product of the tobacco industry. Extraction and purification procedures are generally well-known in the tobacco industry. As has been described, nicotine is colorless to pale yellow in color and is characterized by being oily and very hygroscopic. Nicotine turns the familiar brown color on exposure to light or air.

It has been found through experimentation that the simple nicotine molecule, such as produced by the nicotine vapor mentioned hereinabove, will typically be absorbed by the buccal mucosa in the mouth and thus be exposed to the rigors of the digestive tract prior to reaching the brain. By complexing the nicotine molecule with another substance a larger, more complex or "enhanced" moiety is formed which will generally resist absorption by the buccal mucosa and be absorbed by the lungs, where the time of transmission to the brain is substantially shortened. Materials which have been found to be useful in the nicotine enhancing step are alcohols, esters, hydrocarbons, aldehydes, ketones and ethers.

Pure nicotin is an alkaline molecule. Because absorption of nicotine vapors is in large part dependent on the pH of the vapors, an acidic material is added to the nicotine mixture. Organic acids have shown general utility in fulfilling this function. Specific acids which have known utility include but are not limited to acetic acid, boric acid, oxalic acid, malic acid, citric acid, ascorbic acid, organic acids, organic acids and combinations of the above.

Finally, a volatile carrier material is added to assist in the general volatility of the nicotine preparation. The preferred volatile carrier material is selected from the group consisting of: acetates, alcohols, menthol, methyl salicylate, aromatic materials and combinations thereof. Many other materials have demonstrated utility as a carrier for the nicotine moiety of this invention.

The nicotine mixture, nicotine enhancing material, organic acid and volatile carrier are mixed in accordance with normal manufacturing procedures. It should be noted in selecting materials that they must be suitable for human exposure and/or consumption. In particular, most chemicals are theoretically toxic if the level of concentration is increased enough. Therefore, it is essential to select the materials for use in the formulation of this invention such that they can be used within accepted toxicity guidelines.

Nicotine (d), nicotine (l), nicotine (dl), and nicotine salts may all be used to advantage in this mixture to provide the nicotine vapor which is inhaled by the user. Ninety-eight percent nicotine (l), a product obtained by Eastman Chemical, stock number 1242, has been used in one embodiment of the device and found to perform with satisfactory results. A preparation of nicotine hydrochloride has also been successfully employed. A number of other materials have been found to provide advantageous results when added to the nicotine mixture. Orange oil obtained from commercial orange extract, or similarly obtained lemon oil, enhance the flavoring of the vapor produced from the nicotine formulation and added to the nicotine mixture. Such flavoring may also be added in the form of synthetic ingredients. Other flavors which may advantageously be used in the cigarette include spearmint, peppermint, and cinnamon.

Menthol may also be added to the mixture for a variety of reasons including flavoring, as a carrier material or to suit the particular taste of the user. The menthol which has been used in U.S.P. Levorotatory, obtained from the Gentry Corporation, and is dissolved in ethanol to form a liquid. In addition, water may be added to the nicotine formulation. The water vapor which thus evaporates into the air reduces the slight feeling of dry mouth which may be experienced after prolonged use of a dispensing device without the addition of such water.

Thus, it can be readily appreciated that the design of this invention is to create a nicotine preparation or formulation, the terms being used interchangeably, to create a nicotine vapor for inhalation by users of traditional combustible cigarettes and the like. In designing this formulation, it has been designed to be adaptable for use in any of a variety of dispensing devices. The particular device used to dispense the nicotine preparation of this invention is not considered part of this invention and is not limiting thereon.

The particular physical form of the preparation of this invention can easily be adapted to suit the particular device used for dispensing the formulation. The viscosity and concentration of the formulation may be varied depending on the particular device being used to administer the nicotine.

The formulation of this invention requires specifically four ingredients. First, a nicotine containing composition must be included. As has been explained previously, nicotine is a clear transparent liquid which is readily vaporized.

In order to modify the pH of the final product to make it more readily absorbable, a pH adjustment means is used. This pH adjustment means typically is an organic acid. The organic acids which have shown or exhibited utility in the formula of this invention include, but are not limited to acetic acid, citric acid, ascorbic acid, boric acid, malic acid, and any other organic acids which are suitable for human consumption.

A significant problem with previous formulations and devices has been the inability to have the substantial portion of the inhaled nicotine molecules pass by the buccal mucosa in the mouth and into the lungs. The reason this has not been possible in the past is the natural nicotine molecular complexes found in the vaporized nicotine are more readily absorbed by the first available absorption media, such as the buccal mucosa. By complexing the nicotine molecule with a molecule not readily absorbed by the buccal mucosa such as acetates, hydrocarbons, menthols or methyl salicylate, it is possible to form a more complex chemical mixture, which for purposes of this application, will sometimes be referred to as a nicotine moiety which as an enhanced ability to pass beyond the buccal mucosa.

The enhanced nicotine moiety will typically bypass the buccal mucosa and make its way into the lungs where it can be absorbed by lung tissue and therefore be passed more directly into the bloodstream flowing directly to the brain. The pleasurable effect of this is more dramatic than if the nicotine is absorbed through the buccal mucosa and forced to go through the digestive system prior to transmission to the bloodstream and eventually the brain. This represents a significant improvement over prior art attempts to formulate nicotine dispensing devices. One of the primary advantages involves the fact that by causing the nicotine to be absorbed by the lung tissue, smaller amounts of nicotine can be used to generate the same pleasurable effect in an individual.

Finally, it is necessary to have a carrier material in which the nicotine can be vaporized. Typical of such materials are menthol, methyl salicylate, and other well known carrier media. Again, it is necessary that the particular carrier material and the concentration of the particular carrier material be appropriate for human consumption.

Additionally, a variety of flavoring materials may conveniently be added to the formulation to provide a desired effect. As has been previously mentioned, well known flavorants which are approved for human consumption, can be used in specified amounts. The use of such flavor materials is not intended to be a limiting factor in this application, but rather, it is intended to recite several of the possibilities for enhancing the final nicotine formulation of this invention.

EXAMPLE 1

A nicotine preparation was prepared by mixing 3.5 grams of nicotine, about 0.2 grams of menthol, about 0.15 grams of acetic acid and about 0.6 grams of Polysynlane. The resulting mixture was then tested for the production of vaporous nicotine in a simulated smoking device such as that illustrated and described in U.S. Patent 4,294,089. The formulation exhibited good complexing ability and a substantial portion of the inhaled nicotine vapor was absorbed by the user's lungs as opposed to the buccal mucosa.

EXAMPLE 2

A nicotine preparation was prepared by mixing 1.75 grams of nicotine, about 0.05 grams of methyl salicylate, about 0.075 grams of acetic acid and about 0.3 grams of Polysynlane. The resulting mixture was subjectively tested in exactly the same fashion as described in Example 1.

0148749

## EXAMPLE 3

A nicotine preparation was prepared by mixing 2 grams of nicotine, 2 grams of menthol, about 0.75 grams of acetic acid, 1 gram of polyphenylmethoxysiloxane (Dow Chemical Stock No. 556), and about 0.045 grams of flavoring obtained from International Flavors and Fragrances, Inc., as Stock No. G3578878. The resulting mixture was again subjectively tested as in the previous examples.

## EXAMPLE 4

A nicotine preparation was prepared by mixing 2 grams of nicotine, 2 grams of polyphenylmethylsiloxane (Dow Chemical Stock No. 556), about 0.015 grams of methylchavicol, about 0.09 grams thymol, about 0.075 grams of acetic acid, and flavorings obtained from International Flavors and Fragrances, Inc., about 0.105 grams of Stock No. G3568864, about 0.075 grams of Stock No. G3578994. Again, the resulting mixture was subjectively tested as in the previous examples.

While specific embodiments of the nicotine preparation are described above, many other variations may occur to those skilled in the art. Our invention includes all those variations which fall within the scope of the appended claims.

CLAIMS:

1.    A nicotine preparation for use in combination with dispensing devices for the non-pyrolytic transmission of nicotine characterized in that there is:

    a.    nicotine;

    b.    an acid for modifying the pH of the preparation;

    c.    a complexing substance which forms a new moiety with said nicotine, and

    d.    a carrier material.

2.    The preparation of claim 1 characterized in that said nicotine is selected from a group consisting of:  nicotine (d), nicotine (1), nicotine (dl), and nicotine salts.

3.    The preparation of claim 1 further including a flavoring material.

4.    The preparation of claim 3 characterized in that said flavoring material is selected from the group consisting of orange flavoring, lemon flavoring, menthol, spearment flavoring, pepperment flavoring, and cinnamon flavoring.

5.    The preparation of claim 3 characterized in that said flavoring material is menthol.

6.    The nicotine preparation of claim 1 further including water in said vaporizable nicotine, said water being included in order to provide for the humidification on nicotine vapors to reduce the drying effect of said vapors on the mouth of the user.

7.    The organic acid of claim 1 characterized in that said organic acid is selected from the group consisting of acetic acid, salicylic acid, oxalic acid, malic acid, ascorbic acid, boric acid, citric acid, organic acids, and combinations thereof.

8.    The preparation of claim 1 characterized in that said organic acid is acetic acid.

9.    The preparation of claim 1 characterized in that said complexing substance is selected from the group consisting of alcohols, esters, hydrocarbons, aldehydes, ketones, and combinations thereof.

10.    The preparation of claim 1 characterized in that said complexing substance comprises polyisobutylene or polysiloxane.

11.    The preparation of claim 1 characterized in that said carrier material is selected from the group consisting of menthol, methyl salicylate, aldehydes, alcohols, esters, ketones, acetates, hydrocarbons, and combinations thereof.

-15-

0148749

12. The preparation of claim 1 characterized in that said volatile carrier material is menthol.

13. The preparation of claim 1 characterized in that said volatile carrier material is methyl salicylate.

14. A nicotine preparation for use in combination with dispensing devices for the non-pyrolytic transmission of nicotine characterized in that there is:

    a. vaporizable nicotine selected from the group consisting of: nicotine (d), nicotine (1), nicotine (dl), and nicotine salts;

    b. an organic acid for modifying the pH of said preparation, said organic acid being selected from the group consisting of acetic acid, salicylic acid, oxalic acid, malic acid, ascorbic acid, boric acid, citric acid, organic acids, and combinations thereof;

    c. a complexing substance which forms a new moiety with said vaporizable nicotine, said complexing substance being selected from the group consisting of alcohols, esters, hydrocarbons, aldehydes, ketones, and combinations thereof, and

    d. a carrier material, said carrier material being selected from the group consisting of menthol, methyl salicylate, acetates, hydrocarbons, and combinations thereof.

15. The preparation of claim 14 further including a flavoring material.

16. The preparation of claim 15 characterized in that said flavoring material is selected from the group consisting of orange flavoring, lemon flavoring, menthol flavoring, spearmint flavoring, peppermint flavoring, and cinnamon flavoring.

17. The preparation of claim 14 further characterized in that there is water, said water being included in said vaporizable nicotine in order to provide for the humidification of nicotine vapor on the mouth of the user.

18. The preparation of claim 14 characterized in that said organic acid is acetic acid.

19. The preparation of claim 14 characterized in that said complexing substance is polyisobutylene or polysiloxane.

20. The preparation of claim 14 characterized in that said carrier material is menthol.

21. The preparation of claim 14 characterized in that said carrier material is methyl salicylate.

22. A nicotine preparation for use in combination with the dispensing devices for the non-pyrolytic transmission of nicotine characterized in that there is:

a. a vaporizable nicotine selected from the group consisting of nicotine (d), nicotine (1), nicotine (dl), and nicotine salts, in combination with water, said water being included in said vaporizable nicotine in order to provide for the humidification of nicotine vapors to reduce the drying effect of said vapors on the mouth of the user;

b. acetic acid to impart an acidic pH to said composition;

c. a polysynlane complexing substance which is co-reactive with said nicotine to form enhanced nicotine molecules, and

d. a methyl salicylate carrier material.


23. The preparation of claim 22 further characterized in that there is a flavoring material.


24. The preparation of claim 23 characterized in that said flavoring material is selected from the group consisting of orange flavoring, lemon flavoring, menthol, spearmint flavoring, peppermint flavoring, and cinnamon flavoring.